# EUROPEAN PATENT SPECIFICATION

(11) **EP 0 654 274 B1**
(45) Date of publication and mention of the grant of the patent: **22.03.2000**
(21) Application number: 94118123.2
(22) Date of filing: 17.11.1994
(51) Int. Cl.: A61L 2/06, A61L 2/24

(54) **Sterilization process and plant for a steam autoclave**
Sterilisierungsverfahren und Anlage für einen Dampfautoklav
Procédé et installation de stérilisation pour un autoclave à vapeur

(30) Priority: 19.11.1993 IT MI932462; 19.11.1993 IT MI932463
(43) Date of publication of application: 24.05.1995
(73) Proprietor: M.O.COM. S.r.L., I-20090 Buccinasco (Milan) (IT)
(72) Inventor: Ongaro, Daniele, I-20090 Pantigliate (IT)
(74) Representative: Lunati, Vittoriano

(56) References cited:
- EP-A- 0 015 328
- EP-A- 0 028 542
- EP-A- 0 177 119
- EP-A- 0 177 123
- EP-A- 0 429 960
- EP-A- 0 492 056
- GB-A- 1 072 100
- US-A- 3 494 725
- US-A- 4 759 909

## Description

The invention relates to a sterilization process and plant for a steam autoclave, to be used in particular in the dental surgery field, as defined in the preamble of the appended Claims 1 and 7.

It is known that steam autoclaves comprise a sterilization chamber for the objects to be sterilized or disinfected, into which distilled water is introduced, which water will be partially converted to steam at high temperatures and pressures, depending on the provisions of the sterilization or disinfection programmes.

In the currently used sterilization programmes, for example temperatures of about 134°C and pressures of about 205 KPa are provided over a period of time of variable duration depending on the nature and porosity of the charge. As far as disinfection is concerned, temperatures close to 105°C are sufficient. Tolerances of one or two centigrade degrees are provided on the above temperature values.

In autoclaves of this kind the sterilizing function is performed by saturated steam carrying out a heat exchange with the material in the sterilization chamber. The air present in the sterilization chamber must be drawn out in order to avoid the formation of air pockets or layers preventing an efficient steam penetration and an appropriate heat exchange between the saturated steam and the material to be sterilized.

In addition air, by its expansion, alters the correct temperature-pressure ratio existing under saturated-steam conditions, thereby giving rise to greater pressures.

Practically, while in the presence of saturated steam alone, pressures and temperatures are closely correlated with each other and reflect well-known theoretical values, in the presence of saturated steam mixed with air said theoretical values are altered and the more the amount of air present is high, the more the pressure is higher than the theoretical saturated-steam pressure at a given temperature.

For the above reasons, in the autoclaves an important air ejection from the boiler chamber is provided prior to the sterilization step.

This is done for example by employing repeated discharges or bleedings of air mixed with steam to gradually reduce the air concentration in the boiler chamber during a pre-conditioning stage, before saturated steam conditions are achieved, as disclosed in EP-A-0 492 056. Air evacuation is made by venting the chamber until a saturated steam condition is sensed.

Another solution for carrying out an almost complete air ejection consists in sucking air from the boiler chamber filled with the material, by means of a vacuum pump, as disclosed in EP-A-0 028 542 and GB-A-1 072 100. To obtain a substantially complete removal of the air, a high vacuum degree is required, for example in the order of about 90% or more.

Taking into account the required vacuum, the vacuum pump must necessarily be a high-delivery pump and, as a result, it will be very expensive and bulky, and will involve energy absorptions that will become increasingly higher with the increase of the achieved vacuum degree.

In addition, this technical solution has the drawback that it does not ensure an appropriate safety level, because an insufficient operation of the vacuum pump may give rise to important temperature unevennesses at the inside of the sterilization chamber.

Another drawback of known autoclaves resides in that the formation of overheated, that is "dry"_{,} steam is likely to occur under some operating circumstances.

Overheated steam produces altered sterilization conditions with respect to the provided ones. Its formation may be due, for example, to the presence of too much material to be sterilized; as a result, there is a great amount of built up heat which causes all the water initially admitted to evaporate.

When the sterilization or disinfection step has been completed, steam is ejected from the boiler chamber the pressure of which therefore rapidly decreases.

Before being drawn from the boiler the sterilized material is submitted to drying in order to eliminate any condensate present on its surfaces.

According to a known technique, drying is carried out by bringing pressure in the sterilization chamber to a value lower than the atmospheric pressure, that is producing a vacuum that will promote the condensate evaporation. Finally a pressure leveling takes place by admitting air into the boiler so as to carry out a pressure compensation, said pressure being brought back again to the atmospheric value.

This drying technique has some drawbacks.

In fact, condensate remains can be found at particular areas, for example in the interstices present in the sterilized objects or materials.

In addition, sometimes steam is not completely ejected, but some steam pockets remain together with the condensate, above all if the material is a great deal relative to the sterilization chamber sizes.

Finally, even when the boiler door has been opened, the sterilized objects and material are still at a high temperature. Therefore it is necessary to let further time elapse before reaching an appropriate cooling.

Under this situation, the technical task underlying the present invention is substantially to eliminate the above drawbacks.

This technical task is substantially achieved by a sterilization process and plant for a steam autoclave, as claimed in Claims 1 and 7.

The description of a preferred embodiment of the invention is now given by way of non-limiting example, with reference to the accompanying drawings, in which:
**Figure 1** is a diagram of the plant of the invention;
**Figures 2a, 2b** show the execution steps of two sterilization cycles, by means of diagrams highlighting the pressure variations during said cycles; and
**Figures 3 to 8** show how some individual operating steps take place, referring to simplified diagrams of the plant.

Referring to the drawings, the process can be put into practice by an autoclave having a boiler shell forming, at the inside thereof, a chamber for objects or material to be sterilized.

The process consists first of all in performing an initial step involving sucking of a first amount of air from the chamber, thereby producing an initial vacuum therein, that is a pressure of a value below the atmospheric pressure, for example corresponding to a vacuum of about 80/85 per cent.

Other values are also possible and at all events the selection of the initially produced vacuum degree must be of a good value although it must not deeply engage the suction members.

In Fig. 2a this initial step is carried out during time t₀-t₁.

Subsequently, a pre-sterilization or intermediate step takes place which comprises a quick admission of a selectively variable water dose into said chamber and gradual heating of said water dose.

The intermediate step is carried out during time t₁-t₂ and the steam generated by water heating is provided to reach, at time t₂, a reference pressure **P**_{**r**} corresponding to the theoretical pressure of the wet saturated steam at the desired sterilization temperature.

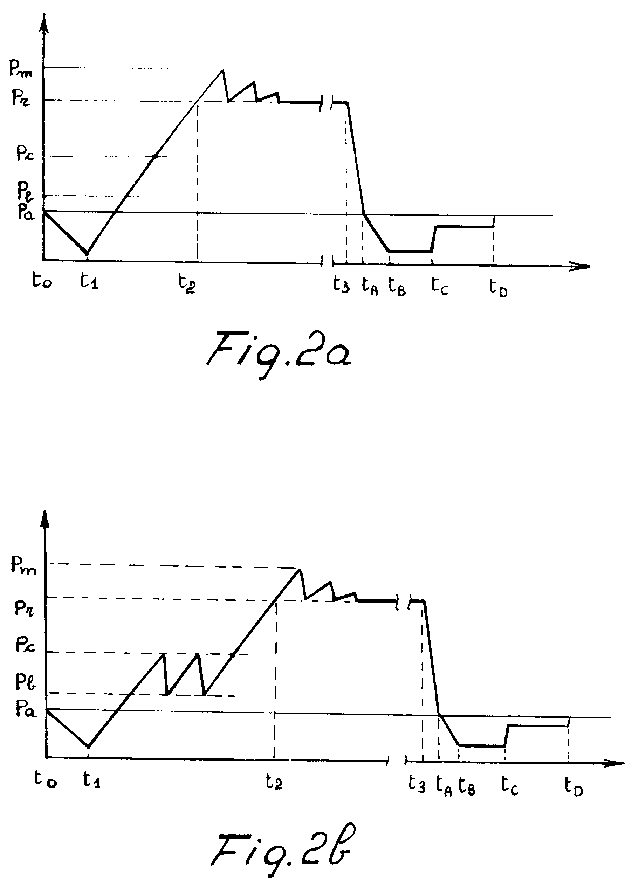

Also provided during the pre-sterilization or intermediate step is an escape or relief stage when the initially reached vacuum degree is lower than approximately 80 per cent. This escape stage consists of one or more strong escapes or drainings obtained by means of timed openings of the sterilization chamber and, as shown in Fig. 2b, is carried out at a control pressure **P**_{**c**} intermediate between value Pᵣ and room pressure **P**_{**a**}.

Practically, the control pressure P_{c} and the corresponding saturated-steam temperature are used as a control threshold conditioning the heating step.

If at the control pressure P_{c} a temperature very close to the theoretical saturated-steam temperature at the same pressure is detected, heating can go on, as shown in Fig. 2a.

If, on the contrary, the detected temperature is greatly lower than the theoretical saturated-steam temperature at pressure P_{c}, said one draining operation is carried out and the sterilization chamber is relieved until a base pressure **P**_{**b**} which is close to the room pressure Pₐ is reached therein, as shown in Fig. 2b. By this draining a second amount of air mixed with steam is discharged.

After draining, pressure starts increasing again, due to the formation of fresh steam and pressure reaches the value of the control pressure P_{c} again.

If, by virtue of the air evacuation obtained by the preceding draining almost steam alone remains in the chamber, on carrying out a new control at pressure P_{c} temperature appears to be close to the theoretical saturated-steam temperature and pressure in the sterilization chamber can rise further.

Should that not be the case, another draining is carried out and optionally other drainings until the achieved temperature value is acceptable.

According to a preferred embodiment of the invention, at least one draining is always done, in order to reduce the preceding suction which in this manner will not be too important, and also to obtain that the following sterilization step shall be carried out in the presence of a minimum amount of residual air.

The control threshold at pressure P_{c} being passed, heating goes on and instant t₂ is reached.

At instant t₂ the sterilization step begins and it goes on until time **t**_{**3**} is reached.

As shown in Fig. 2a, during this step pressure within the sterilization chamber is allowed to grow until it reaches a maximum pressure **P**_{**m**} higher than the reference pressure Pr.

When the maximum pressure Pₘ has been reached, one or more bleedings are carried out, that is controlled drainings similar to the above drainings but of a smaller amount and due to openings of very short duration of a hole formed in the chamber.

Thus a final ejection of a third residual amount of air mixed with steam is caused, and above all a pressure reduction, said pressure being immediately brought to the reference pressure value Pᵣ.

In the case of a permanent tendency of pressure to rise again beyond value Pᵣ, other bleedings are automatically carried out, until in the sterilization chamber the correct reference pressure Pᵣ is set up permanently, at the desired sterilization temperature.

The above bleedings not only enable further air to be eliminated from the sterilization chamber, and pressure to be at all events brought to the ideal value Pᵣ, as already said, but also cause the preceding heating step to be shortened.

In fact, the use of strong heating powers is possible, as well as a quick pressure increase beyond the reference pressure Pᵣ in the chamber, since overpressure is immediately compensated for by means of said controlled bleedings.

It is also to be noted that said controlled bleedings do not hinder the sterilization step and that from instant t₂ the pressure and temperature conditions never go below the minimum ones permitted for sterilization.

In fact, when said controlled bleedings bring pressure to value Pᵣ, temperature remains at all events very close to the ideal sterilization temperature and widely within the tolerance limits. This is due to the fact that, as a result of the initial suction and/or said escape step, pressure Pᵣ can be reached only in the presence of a foreseen satisfactory converging of pressure and temperature values towards the respective theoretical values.

Should the pressure and temperature sensors provided in the boiler signal the presence of overheated steam, that is a complete absence of water in the liquid state, admission of an additional water dose is provided.

When the sterilization step t₂-t₃ is over, a final step, also referred to as drying step, begins, and steam is exhausted to the outside of the boiler chamber so that pressure in said chamber quickly decreases until at instant **t**_{**A**} the atmospheric pressure is reached.

In order to promote evaporation of the condensate present on the surfaces of the sterilized material, that is for the purpose of carrying out drying of said material, according to the process, in said final step a forced vacuum is created which is capable, at instant **t**_{**B**}, of bringing pressure in the sterilization chamber to a value below the atmospheric pressure and maintaining said pressure over a time t_{c} necessary to convert the condensate to steam.

The above forced vacuum is obtained by suction of the residual steam that, being of small amounts, can be also ejected according to suction flow rates lower than those necessary to implement in short periods of time the initial vacuum, which initial vacuum is characterized on the contrary by air ejection.

In addition, according to the process, a forced ventilation step is provided to be carried out within the autoclave consecutively that is starting from time t_{c}, which step involves the formation of an air stream having a conveying direction included between an inlet of filtered air, upstream, and an outlet of air from the autoclave itself, downstream.

The ventilation air stream can be generated, continuing the suction step, by the same means carrying out said forced suction. In addition, the forced ventilation obtained by suction enables the autoclave to be kept under vacuum even during the filtered air admission.

The ventilation step, carried out between instant t_{c} and a subsequent instant **t**_{**D**}, is therefore activated sequentially to the preceding step involving suction alone and enables the evaporation phenomenon already begun by vacuum only, to be increased to an important degree.

In fact, the fresh and sterilized air admitted to the autoclave greatly increases the temperature difference between the still hot sterilized material and the surrounding atmosphere invaded by said fresh air. Consequently, the heat stream transmitted in the time unit from the sterilized material increases, thereby greatly increasing the evaporation amount and speed.

The final or drying step ends as soon as, at instant t_{D} the forced suction is caused to stop; pressure immediately goes to the atmospheric value and the door can be opened.

If the door is not opened, ventilation can be periodically repeated in order to prevent the air humidity from causing the building up of condensate.

The above described steam evacuation method is not applied when liquids are sterilized, in order to avoid said liquids coming to the boil. In this case a gradual cooling is provided without creating any vacuum.

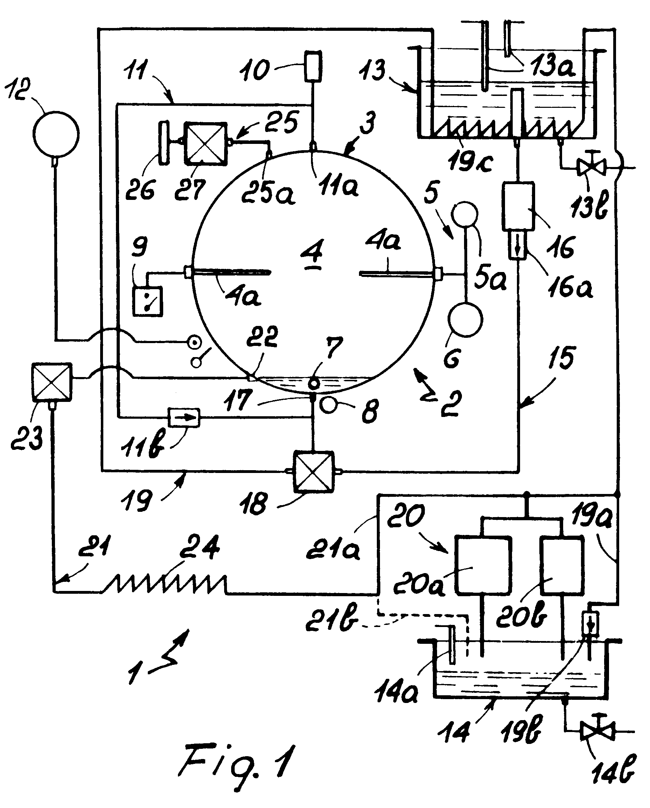

The plant putting into practice the above described process is identified by reference numeral **1** in Fig. 1.

It comprises an autoclave **2** having a boiler shell **3** defining at the inside thereof a sterilization chamber **4** heated by resistors **4a** and accessible through a door, which chamber is designed to house appropriate trays carrying the objects or charge to be sterilized. The autoclave 2 is provided with means **5** for controlling temperature and pressure in chamber 4. In the embodiment shown said means comprises pressure and temperature measuring sensors such as a pressure sensor **5a**, a mechanical pressure meter **6**, a first heat probe **7** at the inside of chamber 4 and a second heat probe **8** at the outside of chamber 4.

Provision is also made for safety devices such as a thermostat **9**, a safety valve **10** disposed close to a surrounding circuit **11** and a microswitch **12** for a magnetic-lag locking of the closing door of chamber 4.

Connection of other different sensors and measurement means and safety devices may be also provided.

In addition, there are control members known per se, of an electronic type for example, acting on all the plant components.

Said surrounding circuit 11 extends from a discharge hole **11a** and is provided with a one-way valve **11b** enabling flows only from the discharge hole 11a.

The plant is further comprised of a feed tank **13** in which distilled water is always maintained between a minimum level and a maximum level, by virtue of waterline detectors **13a**, and a discharge tank **14** having a maximum level detector **14a** for collection of the water used in the sterilization cycles.

Said tanks are provided at the bottom with drain cocks **13b** and **14b.**

A feeding circuit **15** extends between the feed tank 13 and chamber 4 and it comprises water admitting members **16** consisting of a metering pump capable of sending dosed amounts of water from tank 13 to chamber 4, in proportion to the duration of its operation. A one-way valve **16a** is connected close to the metering pump 16 and it prevents return flows to the pump 16.

The feeding circuit 15 opens into the chamber 4 by means of a connecting hole **17** at which the surrounding circuit 11 terminates and is provided with a three-way solenoid valve **18.**

Also connected to the three-way solenoid valve 18 is a discharge circuit **19** comprised, at its ends, of pumping members **20** and a branch **19a** provided with an exhaust or nonreturn valve **19b**.

The discharge circuit 19 upstream of the pumping members 20 passes through the feed tank 13 and is provided, in the vicinity thereof, with a condensate coil **19c**.

Preferably the pumping members 20 are comprised of two vacuum pumps disposed in parallel and different from each other. For example, one of said pumps or main pump **20a** is a two-stage positive-displacement pump, whereas the second or auxiliary pump **20b** is a single-stage pump and preferably has the same delivery as the first one. An exaggerated performance is not required of the pumps in question, as it happened, on the contrary, in the known art, in that reaching of a vacuum degree equal to about 80/85 per cent is sufficient. The second pump may be activated individually or in combination with the first pump.

Extending from the sterilization chamber 4 are bleeding members mainly comprising a bleeding circuit **21** one end of which opens into a bleed hole **22** and the other end of which is connected both to the pumping members 20 and the branch 19a and valve 19b of the discharge circuit 19. The bleeding circuit 21 downstream of the bleed hole 22 comprises a two-way escape and bleed solenoid valve **23** enabling bleedings to be controlled by bringing the hole 22 into communication with the pumping members 20.

Also provided downstream of the two-way solenoid valve is one heat-exchanger **24** for condensing steam coming out of the chamber 4 through hole 22.

Practically the discharge circuit 19 and bleeding circuit 21 extend in parallel starting from the respective holes 17 and 22 and both terminate at the pumping members 20. However, the two circuits 19 and 21 may be maintained separated and it is possible to make circuit 21 directly open into the discharge tank 14, by substituting portion **21b** for its final portion **21a**.

Finally, an air admitting circuit **25** is present and it terminates at an air admitting hole **25a** formed on top of chamber 4. The air admitting circuit 25 comprises a bacteriologic filter **26** through which the external air enters and a two-way compensation solenoid valve **27** controlling air admission.

It will be noted that practically the air admitting circuit 25 and pumping members 20, when combined together in operation, embody ventilation means capable of creating a filtered-air forced circulation within the chamber 4.

Said control members known per se and of the electronic type for example, act on the solenoid valves and pumping members and in particular comprise timing elements adapted to delay stopping of the pumping members relative to the solenoid valve 27 opening for admission of the filtered air.

Operation of the plant is as follows.

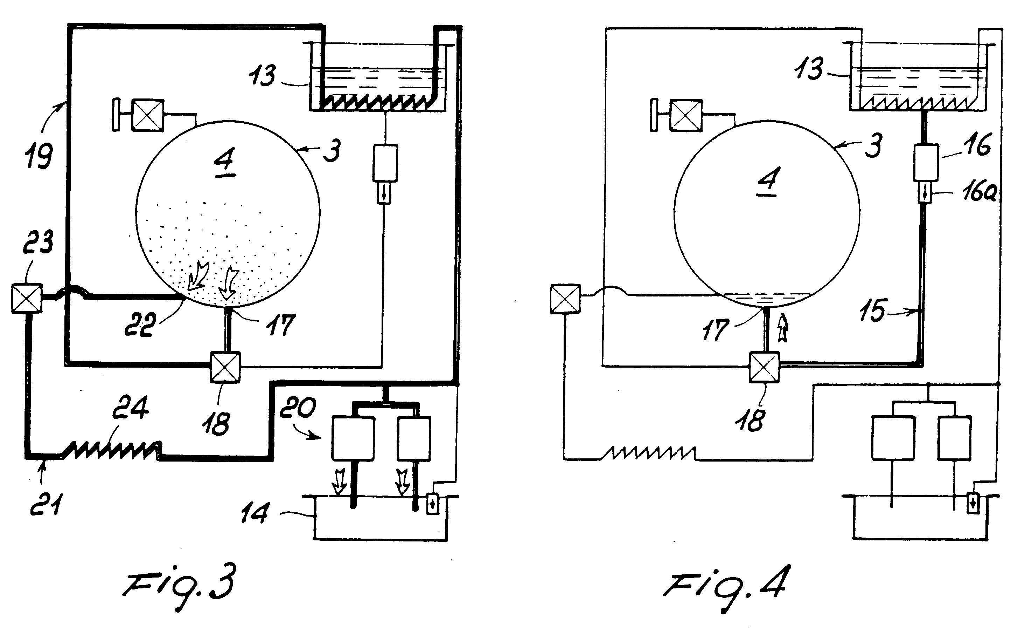

After the autoclave has been charged with the material to be sterilized and the door thereof has been closed, the pumping members 20, and more particularly both the main vacuum pump and the auxiliary pump, are activated, so that the air contained in chamber 4 is sucked quickly and a vacuum corresponding for example to 80/85 per cent is created therein. During this step involving formation of an initial vacuum in chamber 4 (Fig. 3) the solenoid valve 18 makes the hole 17 communicate with the discharge circuit 19 and the solenoid valve 23 is kept open so as to enable air to be drawn also through the bleeding circuit 21. The nonreturn valve 19b prevents air from entering the branch 19a.

Once the foreseen vacuum level has been reached, the solenoid valve 18 closes the communication with the discharge circuit 19 and opens the passage between the feeding circuit 15 and chamber 4. The solenoid valve 23 closes as well, and operation of pumps 20 stops. The metering pump 16 draws distilled water from the feed tank 13 (Fig. 4) and admits it to chamber 4. The dose of the admitted water depends on the duration of operation of the pump.

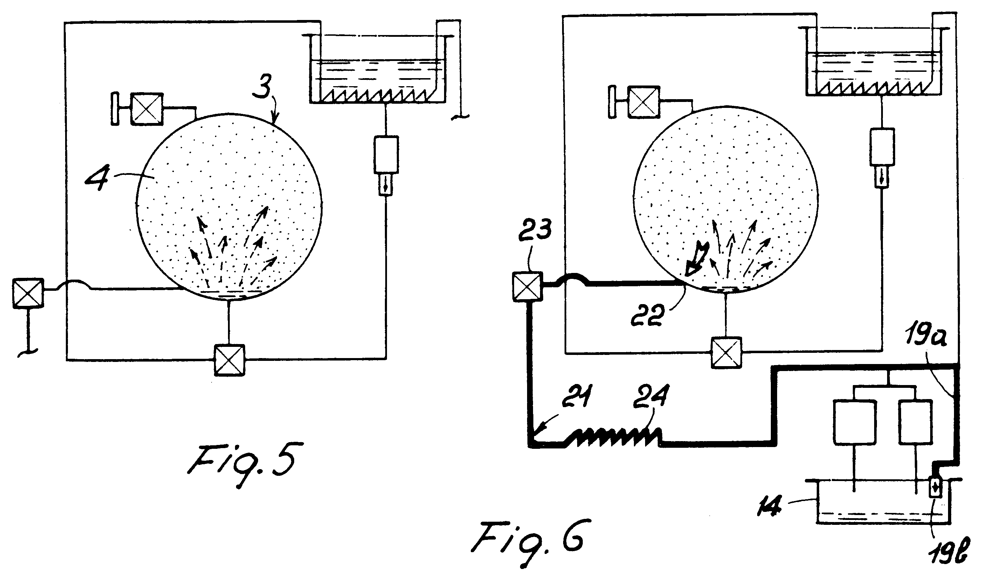

The admitted water is heated by electric resistors and it partially evaporates (Fig. 5) so that within the chamber 4 a temperature higher than the sterilization temperature is gradually reached, for example a temperature of 135°C, as well as a pre-established maximum pressure Pₘ, of about 215 KPa (KiloPascal) for example.

Said maximum pressure Pₘ is higher than a reference pressure Pᵣ corresponding to about 205 KPa which is the theoretical saturated-steam pressure at the ideal sterilization temperature corresponding to about 134°C.

It will be recognized that Pₘ is higher than Pᵣ not only due to the corresponding higher temperature, but also as a consequence of the small amount of residual air still present in chamber 4.

Before said pressures are reached, a draining or relief operation is preferably carried out, at the moment that in chamber 4 a control pressure P_{c} intermediate between the room pressure Pₐ and reference pressure Pᵣ is set up. In the sterilization case, P_{c} is about 100 KPa and draining is prolonged until a base pressure P_{b} of about 50 KPa.

After draining, heating can go on only if at pressure P_{c} the temperature in the chamber 4 is close to the theoretical saturated-steam temperature at the same pressure. Should that not be the case, other drainings are carried out in order to exhaust the air present.

At pressure Pᵣ a series of bleedings is caused to begin and said bleedings are controlled by the solenoid valve 23 and they cause the ejection of the residual air mixed with steam through the bleeding circuit 21, branch 19a and nonreturn valve 19b (Fig. 6). Steam present in the drawn air condensates in the heat-exchanger 24.

The pressure due to bleeding in chamber 4 is each time suitably brought to the reference pressure Pᵣ (Fig. 2a). Bleeding takes place in an automatic manner, as it depends on the temperature and pressure conditions within the chamber 4 and stops, after an appropriate number of bleedings, when pressure, due to a substantial absence of air, stays at value Pᵣ.

Should the plant sensors detect temperature and pressure values indicating the presence of overheated steam in the boiler, due to the absence of residual water, the water admitting members 16 will introduce an additional water dose.

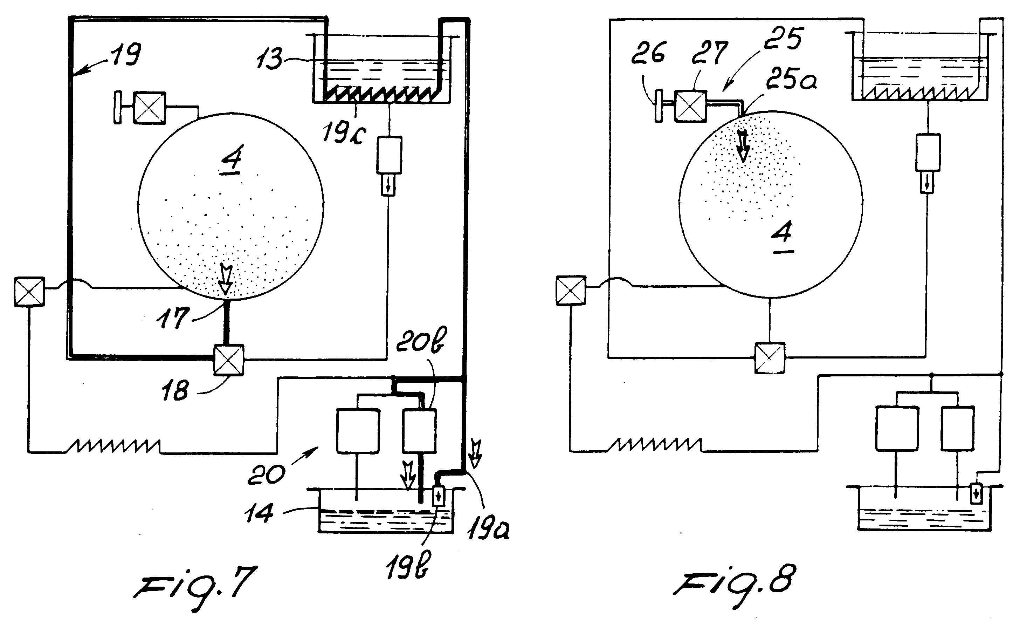

At the end of the process, except for the case in which sterilization involves liquids, steam is evacuated through the discharge circuit 19, the solenoid valve 18 being switched over and the discharge circuit 19 being brought into communication with chamber 4 (Fig. 7).

Steam condensates when passing through the coil 19c and is discharged through the nonreturn valve 19b. Pressure in chamber 4 rapidly drops and when it reaches the atmospheric pressure at time t_{A} or even earlier, the pumping members 20, and in particular the auxiliary vacuum pump 20b alone, are activated and they create a forced vacuum in the chamber 4 itself which is reached at time t_{B}, in order to promote evaporation of the condensate present on the already sterilized material and therefore drying.

The condensate evaporation is completed rapidly, as soon as, at time t_{C}, opening of the solenoid valve 27 is operated, in order to enable admission of the filtered air to chamber 4.

The pumping members 20, and in particular the auxiliary vacuum pump 20b, go on being operated after the beginning of filtered air admission to chamber 4, for an appropriate period of time included between instants t_{C} and t_{D}, which not only enables the chamber 4 itself to be still maintained under vacuum but also creates a strong circulation of air directed towards the outlet hole 17.

The air stream invades all interstices of the sterilized material causing a sudden evaporation of all condensate still present, due to the occurrence of a high thermal head.

The drying step as a result of air circulation practically stops at moment t_{D}, as soon as the pumping members 20 are stopped: pressure evens out to the atmospheric value and the autoclave door can be opened.

If the door is not opened, every ten minutes for example there is activation of the ventilation operation for one minute in order to eliminate the condensate formed by the air itself.

The invention achieves import ant advantages.

First of all the execution of bleedings and drainings in combination with the initial air suction enables said air to be drawn out in several discrete operations. Therefore, the initial vacuum degree can be of a lower amount than in the known art and the operation of the vacuum pumps takes place over a shorter period of time and in addition said pumps absorb less energy. Furthermore, the operations for eliminating air are very reliable as they are based on several steps carried out with different means.

In case of malfunction of the pumps, bleedings and drainings carried out several times ensure at all events that air is properly drawn out. On the contrary, in the case of incomplete bleedings, it is possible to activate the pumps to such a degree that acceptable conditions are created.

Finally, should the pumps, or alternatively the members carrying out bleeding, completely fail, it will be still possible to use the plant temporarily by prolonging to its maximum extent the action of the parts still in operation.

The use of two vacuum pumps enables the delivery of the sucked air to be varied, because at the end of the cycle a delivery corresponding to one pump alone is required. In addition there is an increased safety in operation because, should one of the two pumps fail, the other would be able to create a sufficient degree of vacuum, although in a longer period of time.

It should be noted that it is essential for the plant to be safe because the health of the persons using the sterilized articles may depend on the plant safety.

It is also pointed out that a bleeding operation executed when a high pressure and temperature is reached does not slow down the sterilization step and in addition allows the initial heating to be carried out with greater power and therefore in a shorter period of time, since exceeding of the working pressure is compensated for at once.

Finally, since it is possible to add water to the chamber 4 at any time, the plant functionality is increased because charges of different amounts can be introduced thereinto without causing steam overheating and in addition strong bleedings are allowed being always compensated for by a new formation of steam due to the constant presence of water.

The sterilized material is drawn from the autoclave completely dried on all its surfaces and already at a reduced temperature because the circulation of fresh air also carries out the cooling of same.

In addition, since drying by means of ventilation is very efficient, it is possible to reduce the time for vacuum drying so that a complete drying can be achieved in a very limited period of time. Therefore the overall duration of the sterilization cycle in an autoclave can be reduced to an important extent, thereby increasing the working rates of the autoclave itself.

## Claims

1. A sterilization process for a steam autoclave, comprising:
- a pre-sterilization step including admission of a water dose to said autoclave and heating of said water, which heating leads to the achievement of a reference pressure (Pᵣ) corresponding to the saturated-steam pressure at the desired sterilization temperature,
- a sterilization step carried out after said pre-sterilization step and prolonged in time, and
- a final step in which, once sterilization has occurred, said autoclave is brought back to room pressure (Pₐ),
- the process being characterized in that a suction step is carried out prior to said pre-sterilization step for producing an initial vacuum in said autoclave to evacuate a first amount of air from said autoclave, in that said heating is prolonged until a maximum pressure (Pₘ) higher than said reference pressure (Pᵣ) is achieved,
- and in that during said sterilization step at least one bleeding is carried out which is adapted to eject from said autoclave a residual amount of air mixed with steam so as to bring pressure in said autoclave from said maximum pressure (Pₘ) to substantially said reference pressure (Pᵣ).

2. A process according to Claim 1, wherein said pre-sterilization step comprises an escape stage in which at least one relief or draining is carried out for ejecting air mixed with steam and in which said escape stage is executed starting substantially from a pre-established control pressure (P_{c}) and is prolonged in time until in said autoclave a temperature close to the theoretical saturated-steam temperature is achieved at said control pressure (P_{c}).

3. A process according to Claim 2, in which said control pressure (P_{c}) is substantially intermediate between said maximum pressure (Pₘ) and room pressure (Pₐ) and in which each individual relief in said escape stage is carried out until a base pressure (P_{b}) included between the room pressure (Pₐ) and said control pressure (P_{c}) and close to the room pressure (Pₐ) is achieved.

4. A process according to Claim 1, wherein said final step comprises a forced vacuum carried out by a suction action of a lower delivery than suction in said initial step.

5. A process according to Claim 1, wherein said final step comprises both a forced suction adapted to produce a vacuum in said autoclave, and a subsequent forced ventilation in said autoclave in a manner adapted to promote the residual-condensate evaporation, said forced ventilation being generated by said forced suction in the presence of filtered air entering said autoclave.

6. A process according to Claim 5, wherein said forced ventilation is periodically repeated in the presence of delays in opening said autoclave.

7. A sterilization plant for a steam autoclave, comprising a boiler shell (3) forming a chamber (4) adapted to hold objects to be sterilized, means (5) for controlling the temperature and pressure in said chamber (4), a water feeding circuit (15) extending between said chamber (4) and a feeding tank (13), water admitting members (16) in said water feeding circuit (15), and a bleeding circuit (21) extending between said chamber (4) and a discharge tank (14), said bleeding circuit (21) comprising bleeding members connected to said chamber (4) and adapted to cause a controlled relief or draining from said chamber (4) when said chamber is in condition of at least partial pressurization,
- characterized in that the plant further comprises a discharge circuit (19) extending between said chamber (4) and said discharge tank (14), said discharge circuit (19) comprising pumping members (20) for carrying out suction from said chamber (4),
- and in that said bleeding circuit (21) extends in parallel to said discharge circuit (19).

8. A plant according to Claim 7, wherein said pumping members (20) comprise at least two vacuum pumps (20a, 20b) disposed in parallel to each other, said two vacuum pumps (20a, 20b) including one main vacuum pump (20a) and one auxiliary vacuum pump (20b) which are selectively activated in a manner adapted to vary at least the suction delivery.

9. A plant according to Claim 7 or 8, wherein said pumping members (20) are connected both to said discharge circuit (19) of said chamber (4) and said bleeding circuit (21).

10. A plant according to Claim 7, further having ventilation means adapted to carry out a forced air circulation in said chamber (4), said ventilation means comprising said pumping members (20) and an air admitting circuit (25) leading to said chamber (4).

11. A plant according to Claim 7, wherein provision is made for an air admitting circuit (25) opening into an air admitting hole (25a) in said chamber (4) and comprising a compensation solenoid valve (27) and a bacteriologic filter (26), and wherein control members are provided which are operatively connected to said compensation solenoid valve (27) and to said pumping members (20) and comprise timing elements adapted to operate stopping of said pumping members (20) after said compensation solenoid valve (27) has been opened.

## Patentansprüche

1. Sterilisierungsverfahren und Anlage für einen Dampfautoklav, umfassend:
- einen Vorsterilisierungsschritt, der die Zuleitung einer Wasserdosis in den genannten Autoklav sowie die Heizung solchen Wassers umfaßt, welche Heizung zur Erzielung eines Bezugsdrucks (Pᵣ) bringt, der dem Druck des Sattdampfes bei der gewünschten Sterilisierungstemperatur entspricht;
- einen Sterilisierungsschritt, der nach dem genannten Vorsterilisierungsschritt ausgeführt und zeitlich verlängert ist; und
- einen Endschritt, wodurch - nach Beendigung der Sterilisierung - der genannte Autoklav zurück zum Umgebungsdruck (Pₐ) gebracht wird;
- wobei das Verfahren dadurch gekennzeichnet ist, daß vor dem genannten Voretnkeimungsschritt ein Saugschritt zur Erzeugung eines Anfangsunterdrucks im gennannten Autoklav vorgenommen ist, damit eine erste Menge Luft aus dem genannten Autoklav ausgelassen wird,
- daß die genannte Heizung solange durchgeführt wird, bis ein Maximaldruck (Pₘ) erzielt ist, der höher als der genannte Bezugsdruck (Pᵣ) ist,
- und daß beim genannten Sterilisierungsschritt wenigstens eine Ausräumung vorgenommen wird, die dafür geeignet ist, ein restliches Luft/Dampfgemisch aus dem genannten Autoklav auszulassen, sodaß der Druck innerhalb des genannten Autoklavs im wesentlichen von dem genannten Maximaldruck (Pₘ) bis zum genannten Bezugsdruck (Pᵣ) herabgesetzt wird.

2. Verfahren nach Anspruch 1, wobei der genannte Vorsterilisierungsschritt einen Ausräumungsschritt umfaßt, wodurch wenigstens ein Entwässerungsablaß zum Auslaß vom Luft/Dampfgemisch durchgeführt ist, wobei der genannte Ausräumungsschritt im wesenmtlichen von einem vorbestimmten Kontrolldruck (P_{c}) an vorgenommen und zeitlich solange verlängert ist, bis innerhalb des genannten Autoklavs eine Temperatur nahe der Sattdampfsolltemperatur unter der genannten Kontrolldruck (P_{c}) erzielt ist.

3. Verfahren nach Anspruch 2, wobei der genannte Kontrolldruck (P_{c}) wesentlich ein Mitteldruck zwischen dem genannten Maximaldrick (Pₘ) und dem Umgebungsdruck (Pₐ) darstellt, und wobei jeder Einzelablaß beim genannten Auslaßschritt solange durchgeführt ist, bis ein Grunddruck (P_{b}) erzielt ist, der zwischen dem Umgebunsdruck (Pₐ) und dem genannten Kontrolldruck (P_{c}) und nahe dem Umgebungsdruck (Pₐ) liegt.

4. Verfahren nach Anspruch 1, wobei der genannte Endschritt ein Zwangsunterdruck umfaßt, der mittels eines Ansaugens niedrigeren Durchsatzes als das Ansaugen beim genannten Anfangsschritt erzeugt wird.

5. Verfahren nach Anspruch 1, wobei der genannte Endsschritt sowohl ein Zwangsansaugen, das für die Erzeugung eines Unterdrucks innerhalb des genannten Autoklavs geeignet ist, als auch eine darauffolgende Zwangslüftug innerhalb des genannten Autoklavs derarat umfaßt, daß eine Verdampfung des restichen Kondenswassers gefördert ist, wobei die genannte Zwangslüftung durch das genannte Zwangsansaugen in Anwesenheit gefilterter in den genannten Autoklav eintretenden Luft erzeugt wird.

6. Verfahren nach Anspruch 5, wobei die gennnate Lüftung periodisch bei Verzügen der Öffnung des genannten Autoklavs wiedeholt wird.

7. Sterilisierungsanlage für einen Dampfautoklav, die einen Kesselkörper (3), der eine für die Aufnahme von zu entkeimenden Gegenständen geeignete Kammer (4) bildet, Steuerungsmittel (5) für die Temperatur und den Druck innerhalb der genannten Kammer (4), einen Wasserzuleitungskreislauf (15), der sich zwischen der genannten Kammer (4) und einem Speisungsbehälter (13) erstreckt, Organe (16) zur Wasserzuführung in den genannten Wasserzuleitungskreislauf (15), wie auch einen sich zwischen der genannten Kammer (4) und einem Auslaßbehälter (14) erstreckenden Abzapfkreislauf (21) enthält, wobei der genannte Abzapfkreislauf (21) Abzapforgane enthält, die mit der genannten Kammer (4) verbunden und dafür geeignet sind, einen gesteuerten Ablaß bzw. Entwässerung aus der genannten Kammer (4) dann hervorzurufen, wenn sich die genannte Kammer wenigstens teilweise unter Luftverdichtung befindet,
- dadurch gekennzeichnet, daß die Anlage ferner einen Auslaßkreislauf (19) enthält, der sich zwischen der genannten Kammer (4) und dem genannten Auslaßbehälter (14) erstreckt, wobei der genannte Auslaßkreislauf (19) Pumpenorgane (20) zur Durchführung eines Absaugens aus der genannten Kammer (4) enthält,
- und daß der genannte Abzapfkreislauf (21) parallel dem genanten Auslaßkreislauf (19) verläuft.

8. Anlage nach Anspruch 7, wobei die genannten Pumpenorgane (20) wenigstens zwei parallel zueienander geschaltete Vakuumpumpen (20a, 20b) enthalten, wobei die zwei genannte Vakuumpumpen (20a, 20b) einen Hauptvakuumpumpe (20a) und eiene Hilfsvakuumpumpe (20b) enthalten, die derart wahlweise betätigt sind, daß wenigstens der Absaugdurchsatz verändert wird.

9. Anlage nach Anspruch 7 oder 8, wobei die genannten Pumpenorgane (20) sowohl mit dem genannten Auslaßkreislauf (19) der genannten Kammer (4) als auch mit dem genannten Abzapfkreislauf (21) verbunden sind.

10. Anlage nach Anspruch 7, die ferner Lüftungsmittel hat, die dafür geeignet sind, einen Zwangsluftumlauf innerhalb der genannten Kammer (4) zu bewirken, wobei die genannten Lüftungsmittel die genannte Pumpenorgane (20) und einen Lufteinlaßkreislauf (25) enthalten, die zur genannten Kammer (4) führen.

11. Anlage nach Anspruch 7, wobei ein Lufteinlaßkreislauf (25) vorgesehen ist, der in ein in der genannten Kammer (4) angeordnetes Lufteinlaßloch (25a) einmündet und ein Ausgleichssolenoidventil (27) sowie einen bakteriologischen Filter (26) enthält, und wobei Steuerungsmittel vorgesehen sind, die mit dem genannten Ausgleichssolenoidventil (27) und mit den genannten Pumpenorganen (20) verbunden sind, und Taktgeberelemente enthalten, die dafür geeignet sind, die Abstellung der genannten Pumpenorgane (20) zu bewirken, nachdem das genannte Ausgleichssolenoidventil (27) geöffnet worden ist.

## Revendications

1. Procédé de stérilisation pour un autoclave à vapeur, comprenant:
- une étape de pré-stérilisation comprenant l'admission d'une quantité dosée d'eau audit autoclave et le chauffage de ladite eau, ce chauffage amenant à l'obtention d'une pression de référence (Pᵣ) correspondant à la pression de la vapeur saturée à la température de stérilisation désirée,
- une étape de stérilisation effectuée après ladite étape de pré-stérilisation et ayant une durée prolongée, et
- une étape finale dans laquelle, après avoir effectué la stérilisation, ledit autoclave est amené de nouveau à la pression ambiante (Pₐ), le procédé étant caractérisé en ce qu'on effectue une étape d'aspiration avant ladite étape de pré-stérilisation pour produire un vide initial dans ledit autoclave de manière à évacuer une première quantité d'air dudit autoclave,
- en ce que ledit chauffage est prolongé jusqu'à quand on atteint une pression maximum (Pₘ) plus élevée que ladite pression de référence (Pᵣ),
- et en ce que pendant l'étape de stérilisation il y a la réalisation d'au moins une purge qui est destinée à éjecter dudit autoclave une quantité résiduelle d'air mélangé à la vapeur, de manière à amener la pression dans ledit autoclave depuis ladite pression maximum (Pₘ) jusqu'à essentiellement ladite pression de référence (Pᵣ).

2. Procédé selon la revendication 1, dans lequel ladite étape de pré-stérilisation comporte un stade d'échappement dans lequel on effectue au moins une action d'évacuation ou de drainage pour éjecter l'air mélangé à la vapeur et dans lequel ledit stade est réalisé à partir essentiellement d'une pression de commande pré-établie (P_{c}) et il a une durée prolongée jusqu'à quand dans ledit autoclave on obtient, à ladite pression de commande (P_{c}), une température proche de la température théorique de la vapeur saturée.

3. Procédé selon la revendication 2, dans lequel ladite pression de commande (P_{c}) est essentiellement une pression intermédiaire entre ladite pression maximum (Pₘ) et la pression ambiante (Pₐ) et dans lequel chaque action d'évacuation dans ledit stade d'échappement est effectuée jusqu'à l'obtention d'une pression de base (P_{b}) comprise entre la pression ambiante (Pₐ) et ladite pression de commande (P_{c}) et proche de la pression ambiante (Pₐ).

4. Procédé selon la revendication 1, dans lequel ladite étape finale comporte un vide poussé effectué par une action d'aspiration de refoulement plus bas que l'aspiration dans ladite étape initiale.

5. Procédé selon la revendication 1, dans lequel ladite étape finale comporte soit une aspiration forcée destinée à produire le vide dans ledit autoclave, soit une subséquente ventilation forcée dans ledit autoclave, de manière appropriée à favoriser l'évaporation du condensat résiduel, ladite ventilation forcée étant engendrée par ladite aspiration forcée en présence d'air filtré introduit dans ledit autoclave.

6. Procédé selon la revendication 5, dans lequel ladite ventilation forcée est répétée périodiquement en présence de retards dans l'ouverture dudit autoclave.

7. Installation de stérilisation pour un autoclave à vapeur comprenant un corps de chaudière (3) formant une chambre (4) destinée à contenir les objets à stériliser, des moyens (5) pour contrôler la température et la pression dans ladite chambre (4), un circuit d'alimentation en eau (15) s'étendant entre ladite chambre (4) et un réservoir d'alimentation (13), des éléments d'admission d'eau (16) dans ledit circuit d'alimentation en eau (15), et un circuit purgeur (21) s'étendant entre ladite chambre (4) et un réservoir de décharge (14), ledit circuit purgeur (21) comprenant des éléments purgeurs reliés à ladite chambre (4) et destinés à causer l'évacuation ou drainage contrôlés de ladite chambre (4) quand ladite chambre est en condition de pressurisation au moins partielle,
- caractérisée en ce que l'installation comporte en outre un circuit de décharge (19) s'étendant entre ladite chambre (4) et ledit réservoir de décharge (14), ledit circuit de décharge (19) comprenant des éléments de pompage (20) pour effectuer l'aspiration de ladite chambre (4),
- et en ce que ledit circuit purgeur (21) s'étend parallèlement audit circuit de décharge (19).

8. Installation selon la revendication 7, dans laquelle lesdits éléments de pompage (20) comportent au moins deux pompes à vide (20a, 20b) disposées parallèlement l'une à l'autre, lesdites deux pompes à vide (20a, 20b) comprenant une pompe à vide principale (20a) et une pompe à vide auxiliaire (20b) qui sont activées sélectivement de manière apte à varier au moins le refoulement d'aspiration.

9. Installation selon la revendication 7 ou 8, dans laquelle lesdits éléments de pompage (20) sont reliés soit audit circuit de décharge (19) de ladite chambre (4) soit audit circuit purgeur (21).

10. Installation selon la revendication 7, ayant en outre des moyens de ventilation destinés à effectuer une circulation d'air forcée dans ladite chambre (4), lesdits moyens de ventilation comprenant lesdits éléments de pompage (20) et un circuit d'admission d'air (25) aboutissant à ladite chambre (4).

11. Installation selon la revendication 7, dans laquelle on prévoit un circuit d'admission d'air (25) débouchant dans un trou d'admission d'air (25a) dans ladite chambre (4) et comprenant une électrovalve de compensation (27) et un filtre bactériologique (26), et dans laquelle on prévoit des éléments de commande qui sont reliés de manière opérationnelle à ladite électrovalve de compensation (27) et auxdits éléments de pompage (20) et comportent des éléments de synchronisation destinés à commander l'arrêt desdits éléments de pompage (20) après l'ouverture de ladite électrovalve de compensation.
